# EUROPÄISCHE PATENTSCHRIFT

(11) **EP 1 621 217 B1**
(45) Veröffentlichungstag und Bekanntmachung des Hinweises auf die Patenterteilung: **20.04.2016**
(21) Anmeldenummer: 05023356.8
(22) Anmeldetag: 28.08.2001
(51) Int. Cl.: A61L 27/44, A61L 27/54, A61L 27/34, A61L 29/12, A61L 29/16, A61L 29/08, A61L 31/12, A61L 31/16, A61L 31/10

(54) **Anitmikrobielles Pulver und Material**
Antimicrobial powder and material
Poudre et matériau antimicrobiens

(30) Priorität: 31.08.2000 DE 10043151
(43) Veröffentlichungstag der Anmeldung: 01.02.2006
(62) Teilanmeldung aus: 01971636.4
(73) Patentinhaber: Bio-Gate AG, 90411 Nürnberg (DE)
(72) Erfinder: Bechert, Thorsten, 91301 Forchheim (DE); Steinrücke, Peter, 91052 Erlangen (DE)
(74) Vertreter: Gassner, Wolfgang

(56) Entgegenhaltungen:
- EP-A- 0 650 945
- WO-A-82/01990
- WO-A-84/01721
- WO-A-95/18637
- WO-A-99/26666
- DE-A- 3 228 849
- DE-A1- 3 110 681
- US-A- 4 476 590
- US-A- 4 592 920
- US-A- 4 849 223
- US-A- 5 595 750
- US-A- 5 814 272
- US-A- 5 837 275
- US-A- 5 895 419
- HERIBERT JOSEPH OEL, GERHARD TOMANDL: 'Das Sintern in der Keramik' November 1983, Seiten V. S.1 - V. S.18
- B.H. GÜNTHER: "Metal nanopowders for electrically conductive polymers", INTERNATIONAL JOURNAL OF POWDER METALLURGY, vol. 35, no. 7, 1999, pages 53-58, ISSN: 0020-7535

## Beschreibung

Die Erfindung betrifft die Verwendung eines Silberpulvers.

Aus der EP 0 190 504 ist eine antimikrobielle Zusammensetzung bekannt, welche 5 bis 10 Gew.% Silber enthält. Zur Verbesserung der antimikrobiellen Eigenschaften ist zusätzlich ein hydratisierbares oder ein hydratisiertes Oxid zugesetzt.

Die DE 31 10 681 C2 beschreibt ein Material für Knochenimplantate. Das Material ist aus einem Polymer hergestellt, dem als antimikrobieller Wirkstoff Silberphosphat zugesetzt ist.

Aus der WO 81/02667 ist ein antimikrobielles chirurgisches Implantat bekannt. Dem Implantat ist als antimikrobieller Wirkstoff metallisches Silber zugesetzt.

Die gattungsgemäße WO 82/01990 beschreibt einen Knochenzement auf der Basis von Polymethylmetacrylat als Hauptkomponente, dem als antimikrobieller Wirkstoff 5 Vol.% eines Silbersalzes zugesetzt ist.

Die US 5,837,275 offenbart ein antimikrobielles Material, das u.a. Silberpartikel mit einer Korngröße von weniger als 200 nm enthält. Das Silbergitter weist Gitterstörungen und Fehlstellen auf, um die Freisetzung von Silber-Ionen zu erleichtern. Die Silberpartikel können eine Korngröße bis zu 5 µm erreichen.

Die US 5,595,750 A offenbart eine antimikrobielle Zusammensetzung, bei der aus Bariumsulfat oder aus Zinkoxid hergestellte Partikel mit einer Silber enthaltenden Beschichtung versehen sind.

Aus der WO 84/01721 ist ein mit Silbersulfat oder Silberazetat versehenes Material bekannt. Dieses Material setzt in einer umgebenden Flüssigkeit innerhalb von 24 Stunden eine Konzentration von mehr als 1 µM an Silber-Ionen frei.

Die DE 32 288 849 A1 beschreibt ein Material mit einem Überzug aus Silber. Dem Material ist elementarer Kohlenstoff oder Titan zugesetzt. Der Zusatz soll eine erhöhte Freisetzung von Silber-Ionen in die Umgebung erleichtern.

Die US 4,849,233 offenbart einen Knochenzement, dem etwa 10 Gew.% elementares Silber sowie Titanoxid oder Tantaloxid zugesetzt sind. Der Knochenzement zeichnet sich durch eine hohe Rate der Freisetzung an Silber-Ionen aus.

Die antimikrobielle Wirksamkeit der nach dem Stand der Technik bekannten Materialien ist mit der sogenannten Hemmhofmessung nachgewiesen worden. Die Hemmhofmessung ist z. B. beschrieben in Raad I. et al., J. Infec. Dis. 173 (1996). Dabei wird das zu prüfende Material in ein Nährmedium, z. B. Agar, eingebettet. Wegen der Freisetzung antimikrobiell wirkender Metall-Ionen bildet sich um das Material ein Hemmhof. Die Ausbildung und die Größe eines solchen Hemmhofs ist nach dem Stand der Technik als Anzeichen für die antimikrobielle Wirksamkeit des Materials gewertet worden. Die nach dem Stand der Technik bekannten Materialien haben z.T. den Nachteil, dass sie nur für eine relativ kurze Zeit eine ausreichend hohe Konzentration an Silber-Ionen freisetzen. Deren antimikrobielle Wirksamkeit ist auf diese Zeit beschränkt. Um diesem Nachteil entgegenzuwirken, setzt man nach dem Stand der Technik relativ hohe Mengen an antimikrobiell wirkenden Metallen zu. Das wiederum führt in vivo zu unerwünschten zelltoxischen Effekten.

Aufgabe der Erfindung ist es, die Nachteile nach dem Stand der Technik zu beseitigen. Es soll insbesondere ein Verfahren zur Herstellung eines antimikrobiellen Materials mit verbesserten Eigenschaften angegeben werden. Das Material soll für den Patienten möglichst verträglich sein.

Diese Aufgabe wird durch die Merkmale des Anspruchs 1 gelöst. Zweckmäßige Ausgestaltungen ergeben sich aus den Merkmalen der Ansprüche 2 bis 6.

Bei den aus Silber gebildeten Aggregaten lassen sich die Primärpartikel aufgrund ihrer äußeren Form noch identifizieren. Die Primärpartikel sind miteinander im Wesentlichen über Sinterhälse gebunden. Die Aggregate bilden eine hochporöse Gerüststruktur.

Ein unter Verwendung des Silberpulvers hergestelltes Material zeichnet sich durch eine hervorragende antimikrobielle Wirksamkeit aus. An der Oberfläche des Materials wird eine ausreichend hohe Konzentration an Silber-Ionen zur Verfügung gestellt. Dabei ist die Rate der Diffusion von Silber-Ionen in das umgebende Gewebe besonders gering. Damit bleibt die antimikrobielle Wirksamkeit auf die Oberfläche des Materials beschränkt. Mit dem erfindungsgemäßen Material können z. B. Knochenzemente, Implantate oder auch implantierbare Vorrichtungen, wie Katheter, mit verbesserten antimikrobiellen Eigenschaften hergestellt werden. Es treten keine unerwünschten zytotoxischen Effekte auf. Die antimikrobielle Wirkung des Materials ist besonders lang anhaltend. Der Patient wird pharmakologisch weniger belastet.

Die aus Silber gebildeten Aggregate weisen eine mittlere Korngröße von 1 bis 20 µm, vorzugsweise 10 bis 20 µm, auf. Die Oberfläche der Aggregate beträgt zweckmäßigerweise 3 bis 6 m²/g. Sie können eine Porosität von bis zu 95% aufweisen. Zweckmäßigerweise liegt die Porosität zwischen 70 und 95%. Die vorgenannten Merkmale tragen zu einer gleichmäßigen und zytotoxisch unbedenklichen Abgabe von Silber-Ionen an eine Oberfläche des Materials bei.

Die Aggregate können mittels Inertgasverdampfung und Kondensation, vorzugsweise bei einem Inertgasdruck von 10 bis 100 mbar, hergestellt werden. Das Metall kann neben Silber einen oder mehreren der folgenden Bestandteile aufweisen: Au, Pt, Pd, Ir, Sn, Cu, Sb, Zn. Das Metall weist zweckmäßigerweise einen im Wesentlichen ungestörten Gitteraufbau auf. So wird eine unerwünscht hohe Freisetzung von Silber-Ionen in das umgebende Gewebe vermieden.

In einem Material mit antimikrobiellen Eigenschaften können höchstens 2 Gew.%, vorzugsweise 0,01 bis 2 Gew.%, an Silberpulver bezogen auf das Gewicht eines Matrixmaterials enthalten. Der vorgeschlagene Zusatz an Silber ist relativ gering. Das Material kann preisgünstig hergestellt werden.

Es hat sich weiter als zweckmäßig erwiesen, dass die Aggregate mit dem Matrixmaterial vollständig infiltriert sind. Die Aggregate sind vorteilhafterweise homogen im Matrixmaterial dispergiert bzw. verteilt. Diese Merkmale tragen dazu bei, dass an allen Orten der Oberfläche des Materials stets eine gleich große Menge an Silber-Ionen freigesetzt wird.

Bei dem Matrixmaterial kann es sich um ein, vorzugsweise aus mehreren Komponenten gebildetes, Polymer handeln. Das Polymer kann im wesentlichen Acrylsäure- und/oder Metacrylsäureester enthalten. Als Matrixmaterial eignen sich aber auch andere nach dem Stand der Technik zur Herstellung von Knochenzementen verwendete Matrixmaterialien.

Ein unter Verwendung des Silberpulvers hergestelltes antimikrobielles Material eignet sich zur Herstellung oder auch zur Beschichtung von Implantaten oder einer implantierbaren medizinischen Vorrichtung, z. B. Katheter oder Intratachealtuben. Insbesondere können, z. B. aus Titan oder Keramik hergestellte Hüftgelenksimplantate, Herzklappen, Stents, Kniegelenksimplantate, Zahnfüllungen, Kontaktlinsen oder Intraokularlinsen mit dem vorgeschlagenen antimikrobiellen Material beschichtet oder hergestellt werden.

Zur Herstellung eines Materials mit antimikrobieller Wirkung können die folgenden Schritte durchgeführt werden:
a) Verdampfen und Kondensieren von Metall unter Inertgasatmosphäre, wobei der Druck des Inertgases und die Verdampfungstemperatur so eingestellt werden, dass aus Primärpartikeln mit einer mittleren Korngröße von 10 bis 100 nm bestehende Aggregate sich bilden und
b) Mischen der Aggregate mit einem aushärtbaren Matrixmaterial.

Das vorgeschlagene Verfahren lässt sich relativ einfach durchführen. Es kann damit ein antimikrobielles Material in gleichbleibender Qualität und relativ preisgünstig hergestellt werden.

Nach einem Ausgestaltungsmerkmal werden die Aggregate nach dem Schritt lit. a klassiert. Zweckmäßigerweise wird eine Korngrößenfraktion der Aggregate im Bereich von 1 bis 20 µm, vorzugsweise 10 bis 20 µm, mit dem, vorzugsweise im flüssigen Zustand vorliegenden, Matrixmaterial gemischt. Die Korngrößenfraktion kann in das Matrixmaterial eingerührt werden.

Es hat sich als zweckmäßig erwiesen, ein Inertgas zu verwenden, das als einen wesentlichen Bestandteil mindestens eines der folgenden Gase enthält: Argon, Krypton, Xenon, Helium.

Wegen der weiteren vorteilhaften Ausgestaltungen wird auf die vorangegangenen Ausführungen verwiesen. Die dort beschriebenen Merkmale können sinngemäß auch beim Verfahren Anwendung finden.

Nachfolgend werden Ausführungsbeispiele der Erfindung anhand der Zeichnung näher erläutert. Es zeigen:
- Fig. 1: die bakterielle Proliferation an Knochenzementen im Vergleich zwischen Silberpulvern nach dem Stand der Technik und dem erfindungsgemäßen Silberpulver,
- Fig. 2: eine rasterelektronenmikroskopische Aufnahme eines Silberaggregats,
- Fig. 3: die Abhängigkeit der Zytotoxizität eines Knochenzements in Anhängigkeit des Gehalts an Silber und
- Fig. 4: einen Hemmhoftest für verschiedene Knochenzemente.

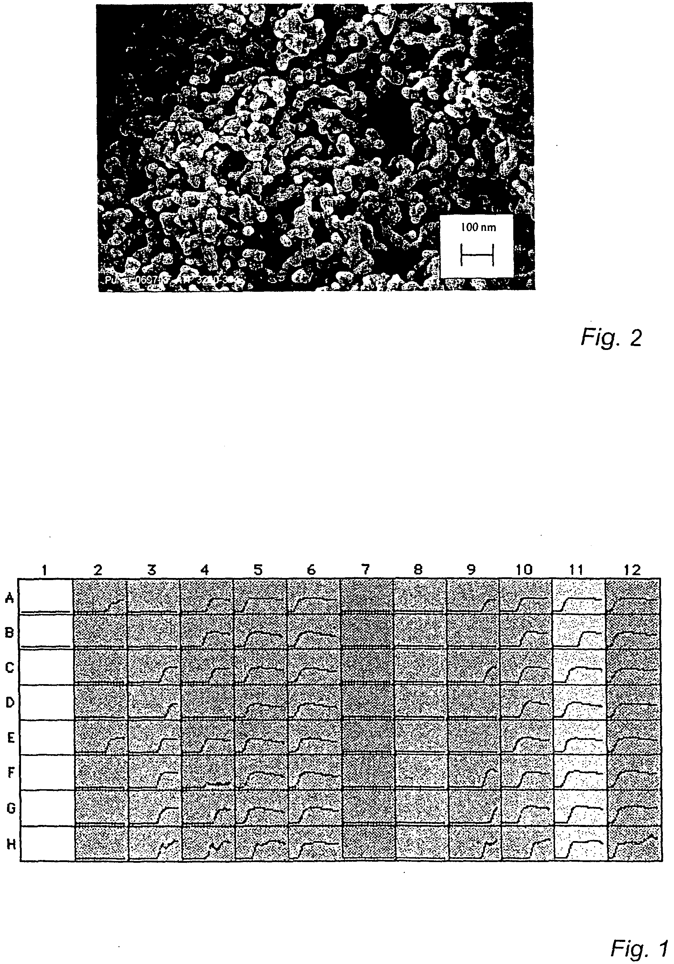

Die in den Fig. 1 gezeigten Ergebnisse sind nach dem aus der DE 197 51 581 A1 bekannten Verfahren ermittelt worden. Dieses Verfahren ist ferner beschrieben in Bechert, Thorsten et al., Nature Medicine, Vol. 6, No. 8 (09/2000).

Es werden zunächst jeweils 8 Parallelproben (A - H) derselben Charge an Knochenzement angefertigt. Die Proben sind üblicherweise zylinderförmig ausgebildet. Sie weisen eine Länge von etwa 1 cm und einen Durchmesser von 2 bis 5 mm auf. Anschließend wird in jede Vertiefung der Mikrotiterplatte 200 µl einer Bakterien-enthaltenden Lösung gefüllt. Die Proben werden bei 37°C für eine Stunde inkubiert. Die Proben werden dann entnommen und dreimal mit physiologischen Puffern gewaschen. Anschließend werden die Proben in die Vertiefungen einer Mikrotiterplatte gelegt, welche mit einem Minimalmedium gefüllt sind. Pro Vertiefung werden 200 µl an Minimalmedium eingefüllt. Die Proben werden für 24 Stunden bei 37°C inkubiert. Anschließend werden die Proben entnommen und verworfen. Zu jeder Vertiefung der Mikrotiterplatte werden 50 µl eines Vollmediums (Trypcasesoja) zugegeben. Anschließend wird die Trübung der Lösung im Abstand von 30 Minuten über einen Zeitraum von 48 Stunden gemessen. Die Lösung wird dabei auf einer Temperatur von 37°C gehalten. Die Trübungsmessung erfolgt mit Licht einer Wellenlänge von 578 nm mittels eines geeigneten Lesegeräts. Eine Trübung zeigt an, dass Bakterien von der Oberfläche der Probe in die Umgebung abgegeben worden sind.

Fig. 1 zeigt einen Vergleich eines Knochenzements, dem unterschiedliche Gehalte an herkömmlichem Silberpulver der Firma Chempur (Spalten 2 - 6) zugesetzt worden sind, mit einem zweiten Knochenzement, dem vergleichbare Mengen an erfindungsgemäßen Silberaggregaten zugesetzt worden sind (Spalten 7 bis 11).

Den Proben der Spalten 2 und 7 sind 2,0 Gew.% Silber, den Proben der Spalten 3 und 8 1,0 Gew.% Silber, den Proben der Spalten 4 und 9 0,5 Gew.% Silber, den Proben der Spalten 5 und 10 0,1 Gew.% Silber und den Proben der Spalten 6 und 11 0,05 Gew.% Silber zugesetzt worden. Spalte 12 gibt die Ergebnisse von Proben ohne Silberzusatz (Kontrolle) wieder.

Es zeigt sich, dass bereits ein Zusatz von 1,0 Gew.% an erfindungsgemäßen Silberaggregaten eine ausgezeichnete antimikrobielle Wirksamkeit zur Folge hat. Bei der Verwendung herkömmlicher Silberpulver wird selbst bei einem Zusatz von 2,0 Gew.% keine zuverlässige antimikrobielle Wirksamkeit erreicht.

Fig. 2 zeigt eine rasterelektronenmikroskopische Aufnahme des erfindungsgemäßen Silberaggregats. Das Silberaggregat besteht im Wesentlichen aus kugeligen Primärpartikeln mit einer mittleren Korngröße von etwa 20 nm. Die Primärpartikel sind im Wesentlichen über Sinterhälse miteinander verbunden. Sie bilden ein hochporöses Gerüst. Das hier gezeigte Silberaggregat hat eine Größe von etwa 10 µm.

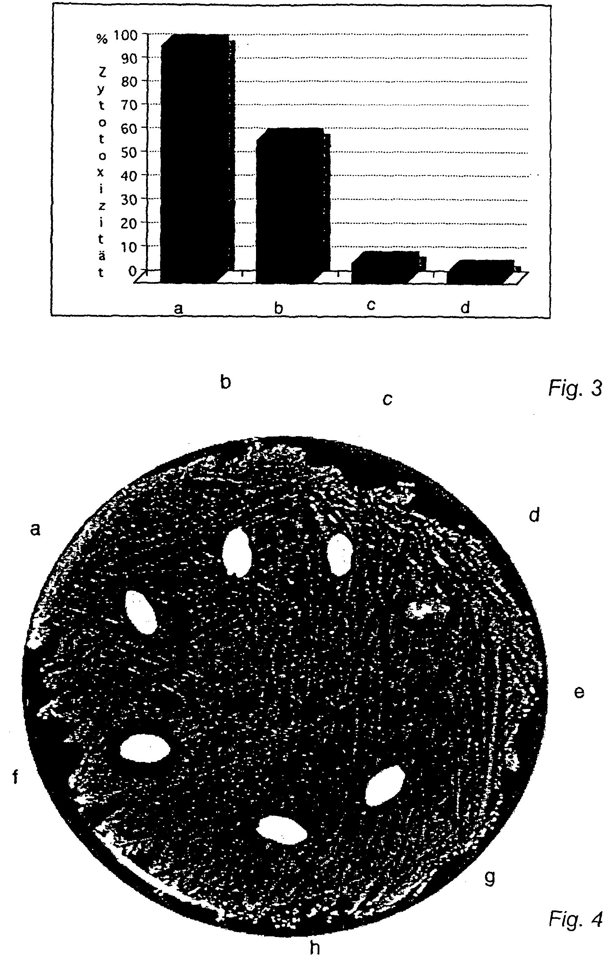

Fig. 3 zeigt die Ergebnisse der zelltoxischen Wirkung der erfindungsgemäßen Knochenzemente. Als Verfahren diente hier der Test nach Greil et al. (Infection, Vol. 27, 1999, Suppl. 1, S. 34 - 37). Dabei wird ein Tetrazolfarbstoff (MTT) durch eine atmungsaktive vitale Zelllinie (MRC-5 Zellen oder durch Polyhämagglutinin stimulierte Lymphozyten) in ein intensiv gefärbtes Formazan umgewandelt. Das Ausmaß der in einem vorgegeben Zeitabschnitt erzielten Verfärbung ist ein Maß für die Vitalität der Zellen. Die Durchführung des Tests erfolgte nach der ISO-Richtlinie. Zunächst werden dazu Extrakte des Knochenzements mit Kulturmedium über 24 Stunden bei 37°C Proben gewonnen. Die Proben werden im Formazan-Assay zusammen mit den Zellen für eine Dauer von 72 Stunden inkubiert. Die Zytotoxizität definiert sich als der in Folge der Extraktzugabe durch Formazanbildung definierte relative prozentuale Verlust der Atmungsaktivität.

Als Positivkontrolle dienten in unabhängigen Experimenten aus PVC entsprechend der ISO-Richtlinie gewonnen Extrakte. Zytotoxizitätswerte größer oder gleich 30% werden als zelltoxische Wirkungen gewertet. Als Kontrollen dienten Extrakte von PE (Negativkontrolle) bzw. PVC (Positivkontrolle).

Fig. 3a zeigt das Ergebnis eines 1:10 verdünnten Extrakts für die Positivkontrolle mit Lymphozyten. Die Zytotoxizität beträgt hier 100%.

Fig. 3b zeigt das Ergebnis der Positivkontrolle mit MRC-5 Zellen. Die Zytotoxizität beträgt hier 60%.

Fig. 3c zeigt das Ergebnis unter Verwendung eines erfindungsgemäßen Knochenzements mit 1,0 Gew.% Silberaggregatzusatz. Die Zytotoxizität betrug hier lediglich 8,4% für Lymphozyten und 4,8% für MRC-5 Zellen (Fig. 3d).

Fig. 4 zeigt die Ergebnisse einer Hemmhofmessung von erfindungsgemäßen Knochenzementen im Vergleich zu herkömmlichen Knochenzementen. Die Probenzusammensetzung war wie folgt:
- Probe lit. a:: Knochenzement mit 0,05 Gew.% an Silberaggregaten,
- Probe lit. b:: Knochenzement mit 0,1 Gew.% an Silberaggregaten,
- Probe lit. c:: Knochenzement mit 0,5 Gew.% an Silberaggregaten,
- Probe lit. d:: Knochenzement mit 2,0 Gew.% an Silberaggregaten,
- Probe lit. e:: Knochenzement mit 5,0 Gew.% an Silberaggregaten,
- Probe lit. f:: herkömmlicher Gentamycin-haltiger Knochenzement (Firma Merck, "Palacos")
- Probe lit. g, h:: herkömmliche Knochenzemente ohne Zusätze (Firma Merck, "Palacos").

Die Proben lit. a bis lit. h sind zur Durchführung der Hemmhofmessung in einen Müller-Hinton-Agar eingebettet worden, der mit Koagulase-negativen Staphylokken als Testkeim für 24 Stunden bebrütet worden ist. Bei den Silberaggregat-haltigen Knochenzementen ist kein Hemmhof erkennbar. Der herkömmliche Gentamycin-haltige Knochenzement zeigt dagegen einen deutlichen Hemmhof. Die erfindungsgemäßen Knochenzemente setzen also nur eine geringe Konzentration an Silber-Ionen in die Umgebung frei.

## Patentansprüche

1. Verwendung eines Silberpulvers, bei dem aus Silber gebildete Aggregate eine mittlere Korngröße von 1 bis 20 µm aufweisen und eine hochporöse Gerüststruktur aus miteinander über Sinterhälse verbundenen Primärpartikeln bilden, wobei die Primärpartikel eine mittlere Korngröße zwischen 10 und 100 nm aufweisen, zur Herstellung eines Materials mit antimikrobieller Wirkung.

2. Verwendung nach Anspruch 1, wobei die Aggregate eine mittlere Korngröße von 10 bis 20 µm aufweisen.

3. Verwendung nach Anspruch 1 oder 2, wobei die Aggregate eine Oberfläche von 3 bis 6 m² pro Gramm aufweisen.

4. Verwendung nach einem der vorhergehenden Ansprüche, wobei die Aggregate eine Porosität von bis zu 95% aufweisen.

5. Verwendung nach einem der vorhergehenden Ansprüche, wobei die Aggregate mittels Inertgasverdampfung und Kondensation, vorzugsweise bei einem Inertgasdruck von 10 bis 100 mbar, hergestellt sind.

6. Verwendung nach einem der vorhergehenden Ansprüche, wobei das Silber einen oder mehrere der folgenden Bestandteile enthält: Au, Pt, Pd, Ir, Sn, Cu, Sb, Zn.

## Claims

1. Use of a silver powder in which aggregates formed of silver have an average particle size of from 1 to 20 µm and form a highly porous framework structure of primary particles bound together by necks formed during sintering, wherein the primary particles have an average particle size between 10 and 100 nm, for producing a material having an antimicrobial effect.

2. Use according to Claim 1, wherein the aggregates have an average particle size of from 10 to 20 µm.

3. Use according to Claim 1 or 2, wherein the aggregates have a surface area of from 3 to 6 m² per gram.

4. Use according to any of the preceding claims, wherein the aggregates have a porosity of up to 95%.

5. Use according to any of the preceding claims, wherein the aggregates are produced by inert gas vaporization and condensation, preferably under a pressure of from 10 to 100 mbar of inert gas.

6. Use according to any of the preceding claims, wherein the silver contains one or more of the following constituent parts: Au, Pt, Pd, Ir, Sn, Cu, Sb, Zn.

## Revendications

1. Utilisation d'une poudre d'argent, pour laquelle des agrégats formés d'argent présentent une taille de grains moyenne de 1 à 20 µm et forment une structure de texture hautement poreuse à partir de particules primaires reliées entre elles par l'intermédiaire de pontages frittés, dans laquelle les particules primaires présentent une taille de grains moyenne comprise entre 10 et 100 nm, pour la fabrication d'un matériau à action antimicrobienne.

2. Utilisation selon la revendication 1, dans laquelle les agrégats présentent une taille de grains moyenne de 10 à 20 µm.

3. Utilisation selon la revendication 1 ou 2, dans laquelle les agrégats présentent une surface de 3 à 6 m² par gramme.

4. Utilisation selon une des revendications précédentes, dans laquelle les agrégats présentent une porosité allant jusqu'à 95 %.

5. Utilisation selon une des revendications précédentes, dans laquelle les agrégats sont fabriqués au moyen d'évaporation sous gaz inerte et condensation, de préférence à une pression de gaz inerte de 10 à 100 mbar.

6. Utilisation selon une des revendications précédentes, dans laquelle l'argent contient un ou plusieurs des constituants suivants : Au, Pt, Pd, Ir, Sn, Cu, Sb, Zn.
